# EUROPEAN PATENT SPECIFICATION

(11) **EP 2 718 704 B1**
(45) Date of publication and mention of the grant of the patent: **27.07.2016**
(21) Application number: 12733222.9
(22) Date of filing: 31.05.2012
(51) Int. Cl.: G01N 27/07, G01N 33/24, G01N 1/14

(54) **PROBE FOR MONITORING THE ELECTRICAL CONDUCTIVITY OF SOIL SOLUTIONS**
SONDE ZUR ÜBERWACHUNG DER ELEKTRISCHEN LEITFÄHIGKEIT VON BODENLÖSUNGEN
SONDE PERMETTANT DE CONTRÔLER LA CONDUCTIVITÉ ÉLECTRIQUE DE SOLUTIONS DU SOL

(30) Priority: 12.06.2011 IL 21349811; 01.03.2012 IL 21843112
(43) Date of publication of application: 16.04.2014
(73) Proprietor: Mottes, Adi, 37105 Karkur (IL)
(72) Inventor: Mottes, Adi, 37105 Karkur (IL)
(74) Representative: Zimmermann, Tankred Klaus
(86) International application number: PCT/IL2012/000213
(87) International publication number: WO 2012/172538

(56) References cited:
- DE-C1- 10 058 416
- FR-A- 1 070 566
- US-A- 4 513 608
- US-A1- 2009 038 390

## Description

### FIELD OF THE INVENTION

The present invention relates to monitoring of chemical and physical properties of soil solutions. More specifically, the present invention relates to a probe and method for direct monitoring electrical conductivity (EC) of soil solution.

### BACKGROUND OF THE INVENTION

The activity and productivity of all agricultural crops are strongly influenced by the level of salinity, i.e. of the total dissolved solids, in the soil solution which is easily determined by electric conductivity measurements

The ability of most crops and plants to draw and extract soil water containing fertilizer by means of the roots depends on the salinity level of the soil solution, i.e., the higher the salinity, or the electrical conductivity, the lower the absorbance of the soil solution by the plant. Therefore, accurate measurement of the level of salinity of the soil solution is decisive in agriculture. Namely, monitoring of the electrical conductivity of the soil solution is highly essential for setting proper treatment scheduling, i.e., for deciding when to irrigate, how much water and fertilizers to use and where they should be applied.

Typical devices for measuring soil properties are disclosed in the following references:
French Patent Publication FR 1070566 discloses an apparatus with an integrated electrical conductivity sensor for taking liquid samples from receptacles/tanks.

US Patent 4,513,608 describes a moisture sensing assembly constructed with different zones of porous material. US Patent 5,418,466 discloses a sensor use for salinity measurement using an oscillating tuned circuit.

US Patent Publication 2009/0038390 discloses a vadose zone probe mounted on an inflatable sleeve.

US Patent Publication 2009/0166520 discloses an in-situ probe having a plunger inserted in the probe.

CN 1362420 relates to a probe with an electrode lead of ion meter connected with ion selective electrode sealed in a porous pottery clay cup in the bottom of probe and connected to an automatic controller via a guide pipe.

The drawback of this probe is the small and narrow inner space of the pottery clay which is not able to hold an EC electrode and to manage electrical conductivity tests.

DE 10058416 discloses a probe and method for determining oxygen content of interstitial water in formations containing sediment and clay. The probe has a very small outer diameter of 5-6 mm and even smaller inner diameter of 2-3 mm which allows the insertion of only a sensor. A vacuum is pulled through the side of the tube. The drawback of such a probe is as follows: the extremely narrow inner diameter of 2-3mm is able to hold only an electrode with very little free space. The very narrow tube may be advantageous for oxygen measurement but not for measuring electrical conductivity. The reason being, that air bubbles enter the tube with soil solution and adhere to the walls of the tube and/or the electrode and in such a narrow tube the bubbles stay there without rising to the surface of the liquid. Such air bubbles distort EC measurement.

The above prior-art references all describe devices and methods for direct monitoring of ground water properties in the soil. These devices and methods may be suitable for monitoring a large number of soil properties such as pH, phosphorus, ammonium, magnesium, chlorides, nitrates, potassium, water hardness, or other soil properties but can produce inaccurate measurements or no readings at all for Electrical Conductivity (EC) since EC is highly sensitive to continuous disturbances in the ground medium. Every change in the volume and direction of the flow of the water as a result of irrigation or rainfall may cause the creation of a temporary physical state of lack of unity in the ground structure. This, in turn, includes the formation of air pockets which disturb the EC measurements. Such situation gets worse when the water content of the ground falls and as a result the electrodes lose contact with the water in the soil.

There is also known the Mottes Soil Solution Extractor and the manual portable testing kits which comprise a probe with a vacuum tube inserted therein, a porous ceramic cap at the bottom and a syringe attached to the vacuum tube at the top. Soil solution is drawn into the probe through the porous ceramic cap by vacuum created by the syringe. Once the soil solution is drawn inside the probe, the solution is withdrawn into the syringe outside of the probe to be checked manually by a portable EC meter and also other testing kits. This probe provides precise in-field testing of soil solution as it changes over time due to fresh soil solution that is drawn in from the soil by the manual syringe operation. The Mottes extractor is accompanied by a variety of portable soil solution testing kits for chloride, nitrate, pH and Electric Conductivity (EC).

Most of the above devices have a porous ceramic section through which ions diffuse in and out of the probe. Such devices may produce correct measurements that are indicative of the true properties of the soil despite the entrapped air in the probe that does not rise to the top surface of the soil solution, and thus, stays in the measuring zone. Air entrapped in the ceramic section space and/or near the electrodes disturbs and restricts the functioning of the EC electrodes and therefore, alters the measurements to be higher than normal resistance readings (lower conductivities, or zero reading).

As noted above, the various available devices that conduct direct monitoring of soil properties within the ground enable the measurements of numerous soil properties except for EC, as EC is highly sensitive to continuous disturbances in the ground solution medium. The principle of measuring the electrical conductivity is based on the electrolytic electrical conduction between two sensor electrodes, spaced apart approximately 6mm. The electrodes are generally sheathed in a rigid or semi-rigid tube, approximately up to 12 cm long. To obtain true EC readings, there must be full and complete solution (liquid) surrounding and between the sensor electrodes without any intervals or disconnections resulting from "air pockets". When the soil is relatively dry, the solution drawn into the porous ceramic tip may lack continuity, and thus, the EC electrodes may not carry out a measurement at all and may show a read error of the value "0".

In sensors for measuring soil properties other than EC, the method of sensing is based on the measurement of an ion concentration in the volume surrounding the sensor, and thus correct measurements can be obtained even if there is no perfect continuity of a solution surrounding the sensor. A pH sensor, for instance, examines the ion concentration of hydrogen in the volume surrounding the sensor.

Thus, the prior art devices and methods are not suitable for conducting EC measurements that are reliable and accurate in all instances, as accurate EC measurements require a perfect continuity of the solution surrounding the sensor electrode.

It is an object of the present invention to overcome or eliminate the problems associated with the prior art devices.

More specifically, it is an object of the present invention to provide a probe that measures in situ, accurately and reliably the electrical conductivity (EC) of soil solutions.

The probe of the present invention is advantageous for obtaining continuous in-situ in-field measurements that are highly precise and reliable. Such measurements provide growers with in-depth information on fluctuations in the electrical conductivity levels which reflect the total dissolved solids in the soil solution. Thus, the growers benefit from water and fertilizer savings, as well as from a good harvest with increased crops together with a reduction in groundwater pollution that keeps the environment clean and green.

### SUMMARY OF THE INVENTION

We have discovered that it is possible to introduce a vacuum tube either through the side or through the top opening of the probe and maintain it parallel with the sensor probe. This allows for continuous monitoring of soil solution, making both, the sensor and vacuum tube an integral part of the probe. Moreover, since the sensor is maintained at all times inside the probe, it is not affected by sunlight, keeping the measurements always stable and reliable.

In accordance with one embodiment of the present invention, there is provided a probe for monitoring electrical conductivity of soil solutions comprising:
an upper hollow tube section stoppered at one end, a lower hollow tube section capped at its bottom with a porous ceramic material for inserting into soil,
a T fitting connecting the other end of the upper hollow tube sections in vertical alignment with the lower hollow tube section, said T fitting having an aperture stoppered,
a sensor probe with an electric conductivity (EC) sensor electrode inserted through the stopper of the upper hollow tube section into the lower hollow tube section, and
a vacuum tube inserted through the stopper of the aperture of the T fitting extending into the lower hollow tube section.

Whereby when the probe is inserted into the soil and a vacuum is applied to the probe through the vacuum tube, ground solution is drawn into the probe through the porous ceramic material to cover the electric conductivity (EC) sensor electrode enabling measuring the electrical conductivity (EC) of the soil solution and
whereby the ground solution can be withdrawn from the probe via the vacuum tube and fresh ground solution drawn into the probe without removing the sensor probe from the probe.

In accordance with the present invention, the aperture in the T fitting is perpendicular to the vertically aligned hollow tube sections.

In accordance with the present invention, the aperture in the T fitting is at an angle to the vertically aligned hollow tube sections.

In accordance with this embodiment of the present invention, the upper and lower hollow tube sections of the probe have outside diameters of between 18 to 25 mm and inside diameters of between 14 to 21 mm to accommodate both the sensor and the vacuum tube and to enable bubbles to rise and not adhere to the inner walls of the tube section, the vacuum tube, or the sensor electrode.

In accordance with another embodiment of the present invention, there is also provided a probe for monitoring electrical conductivity of soil solutions. The probe comprises:
a hollow tube stoppered at its top end and capped at its bottom end with a porous ceramic material for inserting into soil;
a sensor probe with an electric conductivity (EC) sensor electrode inserted into the hollow tube through the stopper at the top end of the tube, and
a vacuum tube inserted into the hollow tube through the stopper at the top end of the tube;
whereby when the probe is inserted into the soil and a vacuum is applied to the hollow tube through the vacuum tube, ground solution is drawn into the hollow tube through the porous ceramic material to cover the electric conductivity (EC) sensor electrode enabling measuring the electrical conductivity (EC) of the soil solution and
whereby the ground solution can be withdrawn from the hollow tube via the vacuum tube and fresh ground solution drawn into the hollow tube without removing the sensor from the probe.

In accordance with the other embodiment of the present invention, the hollow tube of the probe has an outside diameter of between 18 to 25 mm and an inside diameter of between 14 to 21 mm to accommodate both the sensor and the vacuum tube and to enable bubbles to rise and not adhere to the inner walls of the tube, the vacuum tube, or the sensor electrode.

In accordance with the present invention, the probe is up to 15 cm long.

In accordance with the present invention, the vacuum tube reaches down near to the ceramic tip.

In accordance with the present invention, the vacuum tube in the hollow tube is substantially parallel to the sensor probe.

In accordance with the present invention, the vacuum tube is comprised of a semi-rigid section inserted into the probe and a flexible section attached to a vacuum generator.

In accordance with the present invention, the semi rigid section has an outside diameter of about 3 to 4 mm and the flexible section has an inside diameter of about 3 to 4 mm.

In accordance with the present invention, the outside diameter of the sensor tube is 6 mm.

In accordance with the present invention, the porous ceramic material permits free movement of all ions in the ground solution into the tube.

In accordance with the present invention, the vacuum tube is connected to a vacuum generator.

In accordance with the present invention, the vacuum generator is a syringe.

In accordance with the present invention, the syringe is connected to the vacuum tube via a valve.

In accordance with the present invention, the sensor probe is connected to a controller/computer to monitor and transfer of measurement data.

In accordance with the present invention, measurement data can be transferred from the sensor to a computer via cellular and internet systems.

In accordance with the present invention, there is provided a method for monitoring of soil solution properties. The method comprises the following steps:
(1) Providing a probe as defined above,
(2) Inserting the probe into the ground within the range of the irrigation means in proximity of a plant or tree roots system,
(3) Applying vacuum to draw ground solution into the probe to cover the EC sensor electrodes,
(4) Conducting measurements via the EC sensor on said solution, and
(5) Reading and analyzing the resulting data.

In accordance with the present invention, the resulting data received from the EC electrode is sampled, collected and transmitted continuously.

Further aims, features and advantages of the invention will become apparent from the following detailed description taken in conjunction with the following figure.

### BRIEF DESCRIPTION OF THE DRAWING

Fig. 1 illustrates one embodiment of a probe for direct monitoring of soil solution properties in accordance with the present invention.
Fig. 2 illustrates another embodiment of a probe in accordance with the present invention.
Fig. 3 illustrates yet another embodiment of a probe in accordance with the present invention.

### DESCRIPTION OF THE PREFERRED EMBODIMENT

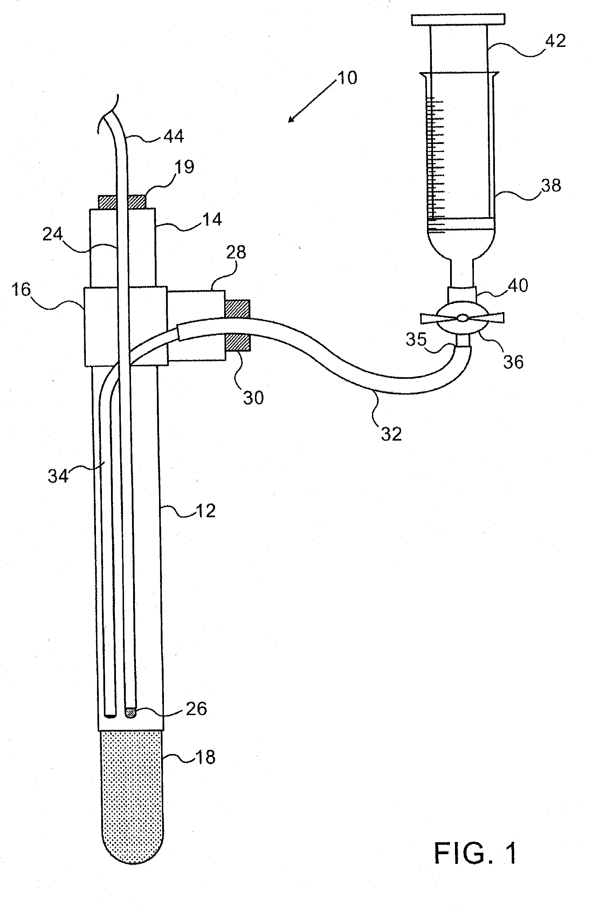

Referring to Figure 1 there is shown a probe 10 comprised of a divided hollow tube consisting of a lower tube section 12 and an upper tube section 14. The two sections 12 and 14 are connected in series through a T fitting 16 by gluing them into the T fitting 16 hermetically. The lower tube 12 section is capped at the bottom with a porous ceramic tip 18 for inserting into soil. The upper tube section 14 is sealed at the top with a stopper 19 which has an opening through which a sensor probe 24 having a sensor electrode 26 at its tip is inserted down towards the bottom of tube section 12.

The T fitting 16 has a side aperture from which extends a hollow tube section 28 sealed (i.e., glued) therein hermetically. Tube section 28 is sealed with a stopper 30. A vacuum tube 32 is inserted through stopper 30 and is connected hermetically to a semi-rigid inner vacuum tube 34 that continues into the lower tube section 12. The other end 35 of the vacuum tube 32 is connected to a valve 36 that is connected to a syringe 38 through its conical tube section 40.

The semi-rigid inner vacuum tube 34 preferably has an outside diameter of about 3mm - 4mm, and the flexible outer vacuum tube 32 has preferably an inside diameter of about 3mm to 4mm.

Such tubing arrangement is suitable since the inner vacuum tube 34 can neither be too flexible nor too rigid in order to maneuver the angle through the T fitting 16 if the inner vacuum tube 34 is to be inserted substantially down into the lower tube 12. The outer vacuum tube 32 is preferable flexible for easy manipulating and positioning of the syringe.

A plunger 42 in the syringe 38 can pull a vacuum in the probe 10. It is preferable that the inner vacuum tube 34 be inserted till the bottom of tube section 12. This will ensure that the entire ground solution drawn into probe 10 can be withdrawn and that fresh ground solution can be sucked in.

The sensor probe 24 has the tip of the sensor electrode 26 located parallel to the inner vacuum tube 34 in tube section 12 preferably extends its entire length (generally 12 cm) down the tube section 12, (but may be shorter as long as it can be immersed in the soil solution). In this embodiment, the probe 10 is relatively short, about 15 cm, and the sensor electrode 26 extends close to the top of the ceramic tip 18.

The sensor electrode 26, in accordance with this invention, is an electrical conductivity (EC) sensor. Electrical conductivity measurement is very sensitive, and unlike measurements such as pH, chlorides, oxygen, nitrates, phosphorus, ammonium, water hardness, potassium, magnesium, or other soil solution properties that are not sensitive to the presence of air bubbles in the liquid media. EC measurements could not be carried out if the liquid media contains air bubbles that may accumulate around the sensor. Such air bubbles are introduced by drawing in the ground water through the ceramic cap 18. Therefore, to provide a soil solution free of air bubbles, tube section 12 preferably has an inner diameter wide enough to enable bubbles to rise to the top surface of the solution and not adhere to the inner walls of the tube section 12, the vacuum inner tube 34 and around or between the sensor electrode 26 itself, which would interfere with the EC measurement. Moreover, the tube section 12 should be sufficiently wide to accommodate both the sensor and the vacuum tube. Therefore, in accordance with the present invention, tube sections 12 and 14 preferably have an outside diameter of between 18-25 mm and an inside diameter of between 14-21 mm in order to accommodate the sensor probe 24 and inner vacuum tube 34 side by side. In a preferred embodiment the sensor probe 24 is connected to a controller (not shown) via cable 44.

The porous ceramic cap 18 is made of inert material, which is not affected by soil components such as fertilizers and allows free transfer of the ions present in the ground solution into the probe 10.

The stoppers 19 and 30 can be made of rubber, plastic, cork, glass or other material able to form a tight seal. The T fitting 16 must be made form a tight fit material to match hermetic sealing (gluing) with tube sections 12, 14 and 28. The syringe 38 should preferably be of sufficient volume (not less than 60 ml.) in order be able to create a vacuum of up to 0.9 bars easily.

The role of the syringe 38, in accordance with the present invention, is two-fold: (1) to create a vacuum and draw soil solution into probe 10 to at least cover the sensor electrode 26 by opening the valve 36 and pulling the plunger 42 fully all the way out and closing the valve 36, then after measuring the electrical conductivity (EC) of the solution, and (2) to transfer the soil solution from tube section 12 to the syringe 38 by opening the valve 36 and drawing the solution from the tube into the syringe 38 while drawing in new fresh soil solution into probe 10 for the next measurement, then closing the valve 36 and expelling the solution from the syringe 38. Thus, filling and emptying of probe 10 with a refreshed ground solution can be achieved quickly and without requiring a significant period of time for the soil solution to come to equilibrium.

It should be noted that the sensor probe 24 is inserted through the stopper 19 of upper tube section 14 into tube section 12 sufficiently to have the sensor electrode 26 completely immersed in the soil solution drawn into the probe 10. It is desired to keep the diameter of the tube section 12 within the limits as defined, in order to draw in soil solution and avoid air bubbles that interfere with EC measurements.

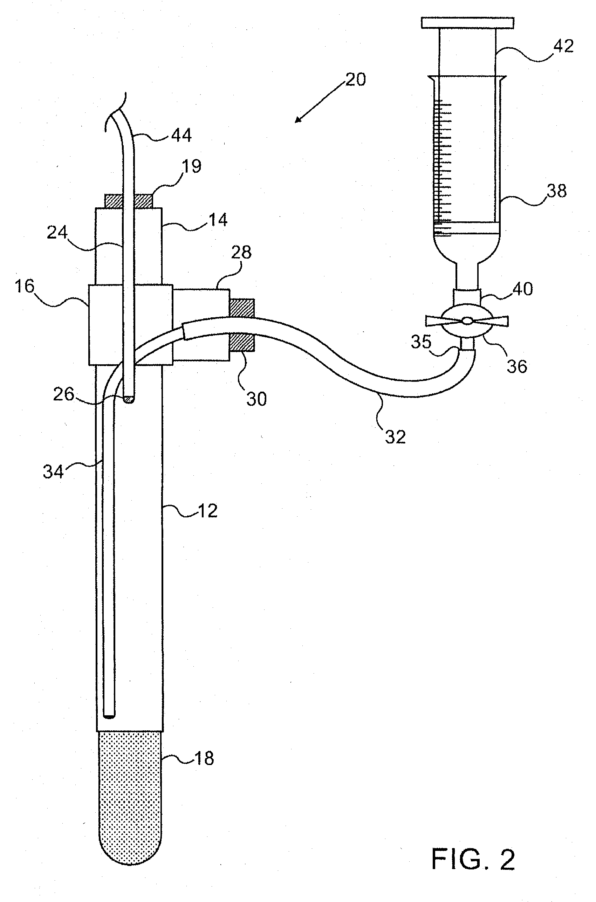

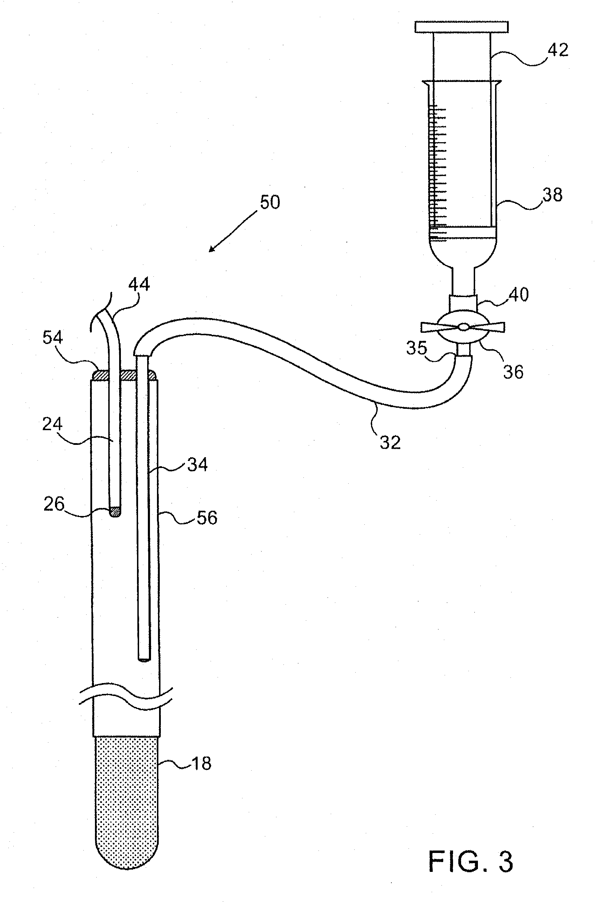

Figure 2 illustrates another embodiment of a probe 20 in accordance with this invention. In this case the electric conductivity (EC) sensor probe 24 does not go to the bottom of the tube section 12, but still can measure the electric conductivity of the soil solution since the vacuum pulled via the inner vacuum tube 34 will bring the solution up in the tube section 12 and cover the sensor electrode 26, enabling correct measurement. The vacuum created by the plunger 42 via the syringe 38 is preferably up to about 0.9 bar as the probe 10 is long and the soil solution is to be drawn up high. Figure 3 shows yet another embodiment of a probe 50, wherein both, the inner vacuum tube 34 and electric conductivity (EC) sensor probe 24, are introduced through a stopper 54 into a tube 56. As can be seen in Figure 3, the probe 50 itself can vary in length depending on the particular soil to be measured, but since the (EC) sensor probe 24 is generally only about 12 cm long, the soil solution is drawn up high enough in the probe to completely cover the sensor electrode 26. The inner vacuum tube 34, however, can be as long as desired; the closer it is to the bottom of the tube the easier to remove all of the soil solution from the tube 56 by vacuum.

### OPERATING PROCEDURES

The operation will be discussed with reference to Fig. 1. The probe 10 operates as follows: the probe 10, having a suitable length for the specific soil depth is inserted into the soil to the depth desired. Initially, the syringe 38 is connected to valve 36 at its connector 40 while the plunger 42 is pushed in fully to the end. The valve 36 is then opened to provide a free air path from the syringe 38 through the vacuum tubes 32, 34 to the inner space of the probe 10. The plunger 42 is then fully withdrawn to create a vacuum through tubes 32, 34 in the probe 10 and the valve 36 is then closed to block the path between the syringe 38 and the vacuum tube 32, in order to maintain the vacuum in probe 10. The syringe 38 may be disconnected from the valve 36 at this time. The soil solution is thus drawn into tube section 12 through the porous ceramic tip 18.

Since the soil solution rises up at the tube section 12, it covers the sensor electrode 26 and measurement can be made continuously.

It should be noted that the syringe 38 as such, is not part of probe 10, but a practical means of creating a vacuum, and can be replaced by other vacuum generators.

After rain or irrigation, the relative concentrations of nutrients, fertilizers, etc. in the ground solution may vary significantly, therefore, one way of measuring electrical conductivity (EC) manually on site is as follows:
(a) The syringe 38 is connected to the valve 36 with the plunger 42 pushed in fully to the end.
(b) The valve 36 is set to an open position between the syringe 38 and the vacuum tubes 32, 34.
(c) The plunger 42 is pulled back all the way to create a vacuum, drawing in soil solution into the tube section 12.
(d) The valve 36 is then closed to block the path between the syringe 38 and the vacuum tube 32, in order to maintain the vacuum in probe 10.
(e) The syringe 38 may be disconnected from the valve 36 at this time, and the plunger 42 pushed back into the syringe 38.
(f) The above procedure may be repeated in order to increase the vacuum in the probe 10 up to about 0.9 bars.

The solution drawn into the tube section 12 is a representative sample of the ground solution and the first measurement may be conducted once the soil solution reaches above the sensor electrode 26.

As time passes, the amount of nutrients, fertilizers and amounts of water in the ground vary and the ions will flow in and out the tube 12 till equilibrium is established between the ions in the ground and the ions in the tube 12. Continuous monitoring of the solution in the probe 10 will provide a curve of the actual fluctuation of ion concentration in the ground solution over time.

In order to improve the equilibrium process, it is recommended to refresh the solution in probe 10 by repeating the above manual procedure, preferably once a week.

The probe 10 is suitable and will work properly in field capacity state and less so in dry soil conditions.

The in-situ measurements may be transmitted continuously from the EC sensor probe 24, via a cellular data-logger to an internet database server for a continuous viewing of the data which may be presented at the website in the form of a graph or table, and for generating analyses of the obtained data based on which the farmer may regulate the delivery and supply of fertilizers, nutrients and irrigation.

## Claims

1. A probe (10) for monitoring electrical conductivity of soil solutions comprising:
an upper hollow tube section (14) stoppered at one end,
a lower hollow tube section (12) capped at its bottom with a porous ceramic material (18) for inserting into soil,
a T fitting (16) connecting the other end of the upper hollow tube sections (14) in vertical alignment with the lower hollow tube section, (12) said T fitting (16) having
an aperture stoppered, an electric conductivity sensor electrode (26) inserted through the stopper of the upper hollow tube section (14) into the lower hollow tube section (12), and
a vacuum tube (32,34)
inserted through the stopper of the aperture of the T fitting (16) extending into the lower hollow tube section (12), whereby when the probe (10) is inserted into the soil and a vacuum is applied to the probe (10) through the vacuum tube (32,34) ground solution is drawn into the probe (10) through the porous ceramic material (18) to cover the electric conductivity sensor electrode (26) enabling measuring the electrical conductivity of the soil solution, and whereby the ground solution is withdrawn from the probe (10) via the vacuum tube (32,34) and fresh ground solution drawn into the probe (10) without removing the sensor electrode (26) from the probe (10).

2. A probe (10) in accordance with claim 1, wherein the aperture in said T fitting (16) is at an angle to the vertically aligned hollow tube sections (12,14).

3. A probe (10) for monitoring electrical conductivity of soil solutions comprising:
a hollow tube (56) stoppered at its top end and capped at its bottom end with a porous ceramic material (18) for inserting into soil;
an electric conductivity sensor electrode (26) inserted into the hollow tube (56) through the stopper (54) at the top end of the tube (56), and
a vacuum tube (34) inserted into the hollow tube (56) through the stopper (54) at the top end of the tube (56) ;
whereby when the probe (10) is inserted into the soil and a vacuum is applied to the hollow tube (56) through the vacuum tube (32,34) ground solution is drawn into the hollow tube (56) through the porous ceramic material (18) to cover the electric conductivity sensor electrode (26) enabling measuring the electrical conductivity of the soil solution and whereby the ground solution is withdrawn from the hollow tube (56) via the vacuum tube (32,34) and fresh ground solution drawn into the hollow tube (56) without removing the sensor electrode (26) from the probe (10)

4. A probe in accordance with any one of claims 1-2, wherein the hollow tube sections (12,14) of the probe (10) have outside diameters of between 18 to 25 mm and inside diameters of between 14 to 21 mm to accommodate both the sensor electrode (26) and the vacuum tube (32,34) and to enable air bubbles to rise and not adhere to the inner walls of the tube section, the vacuum tube (32,34) or the sensor electrode (26).

5. A probe (10) in accordance with claim 3, wherein the hollow tube (56) of the probe (10) has an outside diameter of between 18 to 25 mm and an inside diameter of between 14 to 21 mm to accommodate both the sensor and the vacuum tube (32,34) and to enable air bubbles to rise and not adhere to the inner walls of the tube, the vacuum tube, or the sensor electrode (26).

6. A probe in accordance with any one of claims 1 to 5, wherein the probe (10) is up to 15 cm long.

7. A probe in accordance with claim 6 wherein the electric conductivity sensor electrode (26) reaches down to the porous ceramic material (18) of the lower hollow tube section (12,56).

8. A probe in accordance with any one of claims 1 to 7 , wherein the vacuum tube reaches down to the porous ceramic material (18) of the lower hollow tube section (12,56).

9. A probe (10) in accordance with any one of claims 1 to 8, wherein the vacuum tube in the hollow tube is parallel to the electric conductivity sensor electrode (26) and separated there from by at least 2mm.

10. A probe (10) in accordance with any one of claims 1 to 9, wherein the outside diameter of the electric conductivity sensor electrode is 6 mm.

11. A probe (10) in accordance with any one of claims 1 to 10 wherein the vacuum tube (32,34) is comprised of a semi-rigid section having an outside diameter of 3-4mm inserted into the probe (10) connectable to a flexible section having an inside diameter of 3-4 mm that is attached to a vacuum generator.

12. A probe (10) in accordance with any one of claims 1 to 11, wherein the vacuum tube (32,34) is connectable to a vacuum generator.

13. A probe (10) in accordance with any one of claims 1 to 12, wherein the electric conductivity sensor electrode (26) is connectable to a controller/computer to monitor and transfer of measurement data said data being transferred from the sensor electrode (26) to a computer via cellular and internet systems.

14. A method for monitoring the electrical conductivity of soil solutions comprising:
providing a probe (10) in accordance with any one of claims 1-13, inserting the probe (10) into the ground in proximity of a plant or a tree roots system,
applying vacuum to draw ground solution into the probe,
conducting electric conductivity measurements via a sensor on said solution in said probe, and
reading and analyzing the resulting data.

15. A method in accordance with claim 14 wherein the resulting data is transmitted continuously.

## Patentansprüche

1. Eine Sonde (10) zum Überwachen der elektrischen Leitfähigkeit von Bodenlösungen, die folgende Merkmale aufweist:
einen oberen Hohlrohrabschnitt (14), der an einem Ende mit einem Stöpsel versehen ist,
einen unteren Hohlrohrabschnitt (12), der an seinem unteren Ende mit einem porösen Keramikmaterial (18) verschlossen ist, zum Einführen in den Boden,
ein T-Stück (16), das das andere Ende der oberen Hohlrohrabschnitte (14), die sich in vertikaler Ausrichtung mit dem unteren Hohlrohrabschnitt (12) befinden, verbindet, wobei das T-Stück (16) eine mit einem Stöpsel versehene Apertur, eine Sensorelektrode für elektrische Leitfähigkeit (26), die durch den Stöpsel des oberen Hohlrohrabschnitts (14) in den unteren Hohlrohrabschnitt (12) eingeführt ist, und ein Vakuumrohr (32, 34), das durch den Stöpsel der Apertur des T-Stücks (16), das sich in den unteren Hohlrohrabschnitt (12) hinein erstreckt, eingeführt wird, aufweist,
wodurch dann, wenn die Sonde (10) in den Boden eingeführt wird und durch das Vakuumrohr (32, 34) ein Vakuum an die Sonde (10) angelegt wird, eine Bodenlösung durch das poröse Keramikmaterial (18) in die Sonde (10) gezogen wird, um die Sensorelektrode für elektrische Leitfähigkeit (26), die eine Messung der elektrischen Leitfähigkeit der Bodenlösung ermöglicht, zu bedecken, und wodurch die Bodenlösung über das Vakuumrohr (32, 34) aus der Sonde (10) entnommen wird und eine frische Bodenlösung in die Sonde (10) gezogen wird, ohne die Sensorelektrode (26) von der Sonde (10) zu beseitigen.

2. Eine Sonde (10) gemäß Anspruch 1, bei der die Apertur in dem T-Stück (16) in einen Winkel zu den vertikal ausgerichteten Hohlrohrabschnitten (12, 14) vorliegt.

3. Eine Sonde (10) zum Überwachen der elektrischen Leitfähigkeit von Bodenlösungen, die folgende Merkmale aufweist:
ein Hohlrohr (56), das an seinem oberen Ende mit einem Stöpsel versehen ist und an seinem unteren Ende mit einem porösen Keramikmaterial (18) verschlossen ist, zum Einführen in den Boden;
eine Sensorelektrode für elektrische Leitfähigkeit (26), die durch den Stöpsel (54) an dem oberen Ende des Rohrs (56) in das Hohlrohr (56) eingeführt wird, und
ein Vakuumrohr (34), das durch den Stöpsel (54) an dem oberen Ende des Rohrs (56) in das Hohlrohr (56) eingeführt wird;
wodurch dann, wenn die Sonde (10) in den Boden eingeführt wird und ein Vakuum durch das Vakuumrohr (32, 34) an das Hohlrohr (56) angelegt wird, Bodenlösung durch das poröse Keramikmaterial (18) in das Hohlrohr (56) gezogen wird, um die Sensorelektrode für elektrische Leitfähigkeit (26), die eine Messung der elektrischen Leitfähigkeit der Bodenlösung ermöglicht, zu bedecken, und wodurch die Bodenlösung über das Vakuumrohr (32, 34) aus dem Hohlrohr entnommen wird und frische Bodenlösung in das Hohlrohr (56) gezogen wird, ohne die Sensorelektrode (26) von der Sonde (10) zu beseitigen.

4. Eine Sonde gemäß einem der Ansprüche 1 bis 2, bei der die Hohlrohrabschnitte (12, 14) der Sonde (10) Außendurchmesser zwischen 18 und 25 mm und Innendurchmesser zwischen 14 und 21 mm aufweisen, um sowohl die Sensorelektrode (26) als auch das Vakuumrohr (32, 34) unterzubringen und zu ermöglichen, dass Luftblasen aufsteigen und nicht an den Innenwänden des Rohrabschnitts, des Vakuumrohres (32, 34) oder der Sensorelektrode (26) anhaften.

5. Eine Sonde (10) gemäß Anspruch 3, bei der das Hohlrohr (56) der Sonde (10) einen Außendurchmesser zwischen 18 und 25 mm und einen Innendurchmesser zwischen 14 und 21 mm aufweist, um sowohl den Sensor als auch das Vakuumrohr (32, 34) unterzubringen und zu ermöglichen, dass Luftblasen aufsteigen und nicht an den Innenwänden des Rohres, des Vakuumrohres oder der Sensorelektrode (26) anhaften.

6. Eine Sonde gemäß einem der Ansprüche 1 bis 5, wobei die Sonde (10) bis zu 15 cm lang ist.

7. Eine Sonde gemäß Anspruch 6, bei der die Sensorelektrode für elektrische Leitfähigkeit (26) bis hinunter zu dem porösen Keramikmaterial (18) des unteren Hohlrohrabschnitts (12, 56) reicht.

8. Eine Sonde gemäß einem der Ansprüche 1 bis 7, bei der das Vakuumrohr bis hinunter zu dem porösen Keramikmaterial (18) des unteren Hohlrohrabschnitts (12, 56) reicht.

9. Eine Sonde (10) gemäß einem der Ansprüche 1 bis 8, bei der das Vakuumrohr in dem Hohlrohr parallel zu der Sensorelektrode für elektrische Leitfähigkeit (26) ist und zumindest 2 mm von derselben getrennt ist.

10. Eine Sonde (10) gemäß einem der Ansprüche 1 bis 9, bei der der Außendurchmesser der Sensorelektrode für elektrische Leitfähigkeit 6 mm beträgt.

11. Eine Sonde (10) gemäß einem der Ansprüche 1 bis 10, bei der das Vakuumrohr (32, 34) aus einem halbstarren Abschnitt besteht, der einen Außendurchmesser von 3 bis 4 mm aufweist und der in die Sonde (10) eingeführt ist und mit einem flexiblen Abschnitt verbindbar ist, der einen Innendurchmesser von 3 bis 4 mm aufweist und der an einem Vakuumerzeuger befestigt ist.

12. Eine Sonde (10) gemäß einem der Ansprüche 1 bis 11, bei der das Vakuumrohr (32, 34) mit einem Vakuumerzeuger verbindbar ist.

13. Eine Sonde (10) gemäß einem der Ansprüche 1 bis 12, bei der die Sensorelektrode für elektrische Leitfähigkeit (26) mit einer Steuerung/einem Computer zum Überwachen und Übertragen von Messdaten verbindbar ist, wobei die Daten über Zellular- und Internetsysteme von der Sensorelektrode (26) an einen Computer übertragen werden.

14. Ein Verfahren zum Überwachen der elektrischen Leitfähigkeit von Bodenlösungen, das folgende Schritte aufweist:
Bereitstellen einer Sonde (10) gemäß einem der Ansprüche 1 bis 13,
Einführen der Sonde (10) in den Boden in der Nähe eines Pflanzen- oder Baumwurzelsystems,
Anlegen von Vakuum, um Bodenlösung in die Sonde zu ziehen,
Durchführen von Messungen der elektrischen Leitfähigkeit über einen Sensor an der Bodenlösung in der Sonde und
Lesen und Analysieren der resultierenden Daten.

15. Ein Verfahren gemäß Anspruch 14, bei dem die resultierenden Daten fortlaufend übertragen werden.

## Revendications

1. Sonde (10) permettant de contrôler la conductivité électrique de solutions de sol, comprenant:
un segment de tube creux supérieur (14) bouchonné à une extrémité,
un segment de tube creux inférieur (12) recouvert dans sa partie inférieure par un matériau céramique poreux (18) pour introduction dans le sol,
un raccord en "T" (16) connectant l'autre extrémité des segments de tube creux supérieurs (14) en alignement vertical avec le segment de tube creux inférieur (12), ledit raccord en "T" (16) présentant une ouverture bouchonnée, une électrode de capteur de conductivité électrique (26) introduite à travers le bouchon du segment de tube creux supérieur (14) dans le segment de tube creux inférieur (12), et
un tube à vide (32, 34) introduit à travers le bouchon de l'ouverture du raccord en "T" (16) s'étendant dans le segment de tube creux inférieur (12), par lequel, lorsque la sonde (10) est introduite dans le sol et un vide est appliqué à la sonde (10) à travers le tube à vide (32, 34), la solution de sol est aspirée dans la sonde (10) à travers le matériau céramique poreux (18) pour recouvrir l'électrode de capteur de conductivité électrique (26), permettant de mesurer la conductivité électrique de la solution de sol, et
par lequel la solution de sol est enlevée de la sonde (10) par l'intermédiaire du tube à vide (32, 34) et une solution de sol fraîche est aspirée dans la sonde (10) sans enlever l'électrode de capteur (26) de la sonde (10).

2. Sonde (10) selon la revendication 1, dans laquelle l'ouverture dans ledit raccord en "T" (16) se trouve selon un angle par rapport aux segments de tube creux alignés verticalement (12, 14).

3. Sonde (10) permettant de contrôler la conductivité électrique de solutions de sol, comprenant:
un tube creux (56) bouchonné à son extrémité supérieure et recouvert dans sa partie inférieure par un matériau céramique poreux (18) pour introduction dans le sol;
une électrode de capteur de conductivité électrique (26) introduite dans le tube creux (56) à travers le bouchon (54) à l'extrémité supérieure du tube (56), et
un tube à vide (34) introduit dans le tube creux (56) à travers le bouchon (54) à l'extrémité supérieure du tube (56);
par lequel, lorsque la sonde (10) est introduite dans le sol et un vide est appliqué au tube creux (56) à travers le tube à vide (32, 34), la solution de sol est aspirée dans le tube creux (56) à travers le matériau céramique poreux (18) pour recouvrir l'électrode de capteur de conductivité électrique (26), permettant de mesurer la conductivité électrique de la solution de sol, et
par lequel la solution de sol est enlevée du tube creux (56) par l'intermédiaire du tube à vide (32, 34) et une solution de sol fraîche est aspirée dans le tube creux (56) sans enlever l'électrode de capteur (26) de la sonde (10).

4. Sonde selon l'une quelconque des revendications 1 à 2, dans laquelle les segments de tube creux (12, 14) de la sonde (10) présentent des diamètres extérieurs compris entre 18 à 25 mm et des diamètres intérieurs compris entre 14 et 21 mm pour recevoir tant l'électrode de détection (26) que le tube à vide (32, 34) et pour permettre que les bulles d'air remontent et n'adhèrent pas aux parois intérieures du segment de tube, au tube à vide (32, 34) ou à l'électrode de capteur (26).

5. Sonde (10) selon la revendication 3, dans laquelle le tube creux (56) de la sonde (10) présente un diamètre extérieur compris entre 18 et 25 mm et un diamètre intérieur compris entre 14 à 21 mm pour recevoir tant le capteur que le tube à vide (32, 34) et pour permettre que les bulles d'air remontent et n'adhèrent pas aux parois intérieures du tube, au tube à vide, ou à l'électrode de capteur (26).

6. Sonde selon l'une quelconque des revendications 1 à 5, dans laquelle la sonde (10) est d'une longueur de jusqu'à 15 cm.

7. Sonde selon la revendication 6, dans laquelle l'électrode de capteur de conductivité électrique (26) arrive vers le bas jusqu'au matériau céramique poreux (18) du segment de tube creux inférieur (12, 56).

8. Sonde selon l'une quelconque des revendications 1 à 7, dans laquelle le tube à vide arrive vers le bas jusqu'au matériau céramique poreux (18) du segment de tube creux inférieur (12, 56).

9. Sonde (10) selon l'une quelconque des revendications 1 à 8, dans laquelle le tube à vide dans le tube creux est parallèle à l'électrode de capteur de conductivité électrique (26) et séparé de cette dernière d'au moins 2 mm.

10. Sonde (10) selon l'une quelconque des revendications 1 à 9, dans laquelle le diamètre extérieur de l'électrode de capteur de conductivité électrique est de 6 mm.

11. Sonde (10) selon l'une quelconque des revendications 1 à 10, dans laquelle le tube à vide (32, 34) est composé d'un segment semi-rigide présentant un diamètre extérieur de 3 à 4 mm introduit dans la sonde (10) pouvant être connecté à un segment flexible présentant un diamètre intérieur de 3 à 4 mm qui est fixé à un générateur de vide.

12. Sonde (10) selon l'une quelconque des revendications 1 à 11, dans laquelle le tube à vide (32, 34) peut être connecté à un générateur de vide.

13. Sonde (10) selon l'une quelconque des revendications 1 à 12, dans laquelle l'électrode de capteur de conductivité électrique (26) peut être connectée à un contrôleur/ordinateur pour contrôler et transférer les données de mesure, lesdites données étant transférées de l'électrode de capteur (26) à un ordinateur via des systèmes cellulaires et Internet.

14. Procédé permettant de contrôler la conductivité électrique de solutions de sol, comprenant le fait de:
prévoir une sonde (10) selon l'une quelconque des revendications 1 à 13,
introduire la sonde (10) dans le sol à proximité d'un système de racines de plante ou d'arbre,
appliquer un vide pour aspirer la solution de sol dans la sonde,
effectuer des mesures de conductivité électrique par l'intermédiaire d'un capteur sur ladite solution dans ladite sonde, et
lire et analyser les données résultantes.

15. Procédé selon la revendication 14, dans lequel les données résultantes sont transmises en continu.
